# EUROPEAN PATENT SPECIFICATION

(11) **EP 3 003 559 B1**
(45) Date of publication and mention of the grant of the patent: **17.04.2019**
(21) Application number: 14735639.8
(22) Date of filing: 03.06.2014
(51) Int. Cl.: B01L 3/00, B65D 5/20, B65D 5/50, B65D 5/54, B65D 75/14, B65D 81/26

(54) **METHOD FOR MANUFACTURING A FLUID-IMPERMEABLE CONTAINER FOR TRANSPORTING A BIOLOGICAL SAMPLE**
VERFAHREN ZUR HERSTELLUNG EINES FLÜSSIGKEITSDICHTEN BEHÄLTERS ZUM TRANSPORTIEREN EINER BIOLOGISCHEN PROBE
PROCÉDÉ DE FABRICATION D'UN RÉCIPIENT IMPERMÉABLE POUR LE TRANSPORT D'UN ÉCHANTILLON BIOLOGIQUE

(30) Priority: 03.06.2013 GB 201309867
(43) Date of publication of application: 13.04.2016
(73) Proprietor: Mast Group Limited, Liverpool, Merseyside L20 1EA (GB)
(72) Inventor: MCELARNEY, Iain, Backwell North Somerset BS48 3NF (GB)
(74) Representative: WP Thompson
(86) International application number: PCT/GB2014/051696
(87) International publication number: WO 2014/195694

(56) References cited:
- EP-A1- 1 174 350
- WO-A1-2008/146010
- WO-A1-2014/118542
- WO-A2-02/45647
- DE-U1-202007 016 728
- US-A- 3 124 248
- US-A- 5 040 678
- US-A- 5 295 580
- US-A- 6 152 493
- US-A1- 2003 206 227
- US-A1- 2008 023 532

## Description

The present invention relates to a method for manufacturing a fluid-impermeable container containing a receptacle for transporting a biological sample.

The rules and legislation governing the transportation of biological material, such as human waste, tissue, or blood samples or specimens, are becoming increasingly stringent. As a result of the health risk to humans posed by pathogens such as bacteria, viruses and fungi, which may be present in such samples, precautions must be taken to guarantee the safety of those operating in this field.

The health risk to humans posed by certain biological material can be greatly reduced by the type of packaging used to store the sample during transit. Moreover, given the recent rise in self-screening initiatives, such as for bowel cancers and the like, the number of biological samples being collected at home, as well as in clinics, is rising. Such samples are invariably couriered in large quantities to medical institutes or laboratories where various analyses are conducted. It has therefore become necessary to provide effective, albeit easy to operate, packaging that complies, amongst others, with the regulations under UN3373, relating to biological substances being transported for diagnostic or investigative purposes.

Regulations such as UN3373 impose strict requirements on the minimum features that such packaging should possess. Biological material that is known or reasonably expected to contain harmful pathogens must be transported in packaging that includes the following key components:
- A primary receptacle, being a tube, vial or other container made of glass or rigid plastic,
- A secondary packaging encapsulating the primary receptacle and being fluid impermeable, and
- An outer packaging,
in which either of secondary or outer packaging is rigid.

Biological samples are commonly collected in vials or tubes made from glass or rigid plastic. A conventional method of packaging such samples is to place the receptacle in a sealable polythene bag, which is then itself packaged within a rigid sleeve or box. However, in self-screening studies, the multi step nature of such a packaging technique often proves over-complicated for certain people, such as the elderly, resulting in the subsequent failing of the packaging, putting those operating in this field at risk. Packaging techniques that require a multiplicity of different packages are also considerably more expensive.

Another type of packaging for use in this field is the Polymed envelope, which is made from strong vinyl material, and has an opening defined by a sealable metal zipper. The Polymed envelope is designed to be used with additional leak proof packaging. It also suffers from certain structural inadequacies, including an insufficiently rigid outer packaging. Additional packaging is therefore required for biological samples whose transportation is governed by strict regulations. The MiniMailBox was also designed with UN3373-type regulations in mind. However, despite being conveniently sized so as to be postable through a standard letter box, the MiniMailBox is not itself leak proof, meaning that additional interior packaging is required, thereby placing an increased burden on the user to ensure that each component of the packaging is correctly and securely sealed .

WO 02/45647 A2 discloses a mailer for shipping cells contained in a cell culture device, wherein the mailer comprises a containment system that can absorb and contain within the mailer a fluid that may leak from the cell culture device. The containment system comprises a gas-permeable absorbent material adapted to surround the cell culture apparatus; and a liquid impermeable layer which seals the absorbent material so as to contain any fluid released within the mailer.

WO 2008/146010 A1 discloses a package that has two complementary casing-parts having a central, outwards-extending depression for accommodating opposite sides of a container. One of the casing-parts is lined with a super-absorbent material lining. The depression in each casing-part is surrounded by a respective surround. The surrounds have margins, which are stepped in the opposite direction from the depressions, i.e. inwards of the closed condition of the package. With the interposition of the lining, the margins abut when the casing-parts are closed together.

The present invention was devised with the foregoing in mind.

According to the present invention, there is provided a method for manufacturing a fluid-impermeable container containing a receptacle for transporting a biological sample, comprising the steps of:
a. folding a blank to form the container, the container comprising folded sheet material, the folded sheet material being fluid impermeable and defining a base, enclosing side walls, an upper wall, and a container closing member, the upper wall being configured to define an opening to a recess for receiving a receptacle containing a biological sample to be transported, wherein the recess is sealingly enclosed by the container closing member when the container is in a closed configuration;
b. inserting a receptacle into the container;
c. applying a transparent plastic film around the container and receptacle; and
d. applying a unique identifier to the receptacle and/or container through the film by contactless printing, wherein the unique identifier is one or more item of information which is specific to the source of the sample.

In one embodiment of the invention, the sheet material is rigid. The container therefore provides both a fluid impermeable barrier, as well as a rigid outer packaging without the need for multiple types of packaging.

In another embodiment of the invention, the container is sized to be postable through a standard letter box. The container thereby facilitates sampling by the public in self-screening studies.

In yet another embodiment of the present invention, the folded sheet material comprises a fluid impermeable coating. Optionally, the fluid impermeable coating is provided as a laminate coating on the folded sheet material. Lamination of sheet material is an inexpensive process, and confers fluid impermeability characteristics to the resulting container.

In still another embodiment of the present invention, the folded sheet material comprises cardboard. Cardboard sheet material is easily cut and shaped, and confers strength and durability characteristics whilst being at the same time cost-effective.

in yet another embodiment of the present invention, the container closing member comprises an adhesive portion. An adhesive affords a good seal around the recess. Optionally, the adhesive portion does not extend over an area of the container closing member that overlies the opening to the recess when the container is in a closed configuration. Whilst the adhesive portion affords a good seal around the upper periphery of the recess, the area of the container closing member that overlies the opening to the recess when the container is In a closed configuration may not comprise an adhesive. Consequently, a receptacle may not become adhered to the container closing member when the container is in use. This may be achieved by die cutting an adhesive strip. The die cutter may be shaped to correspond to the opening of the recess. Optionally, the adhesive is exposed by releasing a backing strip. The container closing member is therefore simple to operate. The adhesive and sealing characteristics of the adhesive are not compromised in the event of sample spillage.

In another embodiment of the present invention, the container further comprises one or more container reinforcing members. The one or more reinforcing member confer additional strength, rigidity and durability characteristics to the container. Optionally, the one or more container reinforcing members comprises an absorbent. The absorbent may be impregnated within the one or more container reinforcing members. In the event of damage to the primary receptacle, the absorbent prevents the sample from leaking out of the container. Optionally, the container reinforcing member comprises one or more inserts, around which the sheet material of the container is formed. Multiple inserts may be stacked one on top of another and then secured in place once the sheet material is erected around them. Optionally, the one or more inserts are made from a sufficiently rigid foam or from corrugated cardboard. Such materials improve the rigidity of the container whilst offering a degree of absorbency.

In another embodiment of the present invention, the recess comprises a cushioning material. The primary receptacle, which is often manufactured from glass or rigid plastic, is thereby cushioned from impacts during transportation and handling. Preferably, the dimensions of the recess correspond accurately to those of a primary receptacle whose lid portion has been correctly closed. Accordingly, the recess is physically incapable of accommodating a receptacle whose lid has been incorrectly closed, thereby prompting a user to take action. Moreover, providing a recess whose dimensions correspond closely to those of the receptacle being transported prevents the lid, cap or stopper of such a receptacle from becoming detached or dislodged during transit. The risk of spillage or leakage is therefore even further reduced.

In yet another embodiment of the present invention, the recess comprises an absorbent material. In the event of damage to the primary receptacle, the absorbent prevents the sample from leaking out of the container. Optionally, the quantity of absorbent material is sufficient to absorb the entire contents of a biological sample to be transported. The risk of any biological material reaching the outer extremities of the container is thereby significantly reduced. Optionally, the absorbent material is a foam or cotton wool. Such materials provide adequate absorbency characteristics, whilst at the same time affording a degree of cushioning.

In still a further embodiment of the present invention, the recess further comprises a fluid impermeable holder for housing a biological sample to be transported. The holder confers additional fluid impermeability characteristics to the portion of the container being in contact with the primary receptacle. Optionally, the holder is integrally formed within the recess. An additional burden on the user is thereby eliminated. Optionally, the holder is made from plastic. Plastics are typically lightweight, fluid impermeable and inexpensive.

In yet another embodiment of the present invention, the recess further comprises one or more cutaway portions for facilitating access to the recess. The usability of the container for less dexterous persons, such as the elderly, is therefore increased.

In still another embodiment of the present invention, the recess may be substantially larger than the receptacle which it is intended to receive. In such an embodiment, the recess may further comprise receptacle retaining means to retain the receptacle in a given part of the recess, such that container opening means, such as a blade or guillotine, may safely shear through an empty part of the recess. Optionally, the receptacle retaining means comprises a constricted portion. The constricted portion may be formed into a single side wall of the recess. Alternatively, opposing side walls of the recess may be constricted at a given point.

In a further embodiment of the present invention, the container further comprises sample releasing means for accessing the recess other than via the container closing member. The container therefore provides means for accessing the packaged sample once it has reached its destination. The means are located away from the container closing member so that its integrity is not weakened. Optionally, the sample releasing means comprises a perforated release tab complementarily shaped to at least one of the faces of the recess. The tab may be simply removed by tearing along the perforation, thereby exposing one or more faces of the packaged sample. Optionally, the perforated release tab is disposed on the base of the container. Alternatively, the sample releasing means may comprise a line of weakness extending through the container and intersecting the recess along one of its faces. The packaged sample may therefore be accessed by breaking the container over the edge of a flat surface, such as a work top, so as to expose one or the faces of the packaged sample. Alternatively, the sample releasing means may comprise a line of weakness extending through the container and intersecting the recess at any point along its length. Once the container is broken along the line of weakness, a portion of the receptacle may protrude from the recess. Such sample releasing means may prove advantageous when numerous containers are to be opened, by reducing the risk posed by repetitive strain injuries (RSIs).

Also disclosed herein is a kit of parts for transporting a biological sample, the kit manufactured in accordance with the first aspect of the present invention.
Also disclosed herein is a pre-cut and pre-creased blank comprising sheet material which is erectable to form a fluid impermeable container formed by the method of the present invention.

The method of the present invention allows a user to personalise a label directly on the receptacle capable of containing a biological sample with one or more unique identifier. The "user" in this context may for example be a health care organization, which provides the container to a patient (or person taking the sample) so that the patient (or person taking the sample) may place the sample within the container. In a broader context the user may be a person or organization which is involved in the dispatch of the containers. It is important to the user that the container is directed to the correct person, and even more important that, when the container is returned with the sample, its source (e.g. the identity of the patient) is clear.

Thus the present invention allows containers and packaging for biological samples to be mass manufactured and subsequently uniquely personalized so that they are for example identified as being uniquely intended for individual patients and furthermore so that when they are returned the biological sample and any analysis or results are attributed to the correct patient.

This feature of unique identification is particularly useful in combination with the container and packaging of the present invention. In combination, it provides a reliable and cost-effective way of enabling analysis and transport of biological samples and ensuring that samples are correctly identified.

In one embodiment of the invention, the cover flap can be folded back and the receptacle capable of containing a biological sample is visible under the outer plastic wrapping.

The one or more unique identifier may for example comprise any one or more of the following: readable patient data, such as, name, date, number which identifies the patient or subject e.g. health record number or health index number, kit number, and a 1D and/or 2D barcode that also encompasses all or part of the above. For example, two types of identifying information may be used, namely information which is readable by the patient, health care professional, or other person, and information which is computer-readable such as a bar code.

The means for applying the unique identifier comprises contactless printing means. More than one type of printing may be used, for example laser printing to impart certain information and inkjet printing to impart certain other information. The invention is flexible and recognizes that different processes and local environments may be appropriate for different aspects of the identifying procedure.

Contactless printing means may apply one or more unique identifier to personalize the receptacle while already packaged (i.e. after the manufacturing process) as described hereinbefore. The contactless printing can take place through the plastic wrap.

The personalization can therefore be done at a different location, and using different apparatus, compared to the manufacturing process. Contactless printing such as laser printing is particularly suitable for imparting specific information on different units at reasonable speed.

By applying a film around the packaging and receptacle by the manufacturer prior to personalisation by the end user, the present invention acts as a single entity thereby enabling sample tracking processes and visual confirmation of personal identifiers by the recipient whilst removing the possibility of mismatching sample kit components during assembly. This is particularly important for data protection purposes as well as ensuring continuity of patient/sample identity throughout the process.

The present invention therefore is applicable for use by high throughput mail fulfilment processes. Alternatively the personalization may take place on a smaller scale, for example on site at a laboratory, health centre or hospital.

The use of contactless printing to impart information within the packaging is advantageous because it avoids the need for exterior labels. Labels affixed to the exterior of the container may be damaged or weather worn. Nevetheless, such labels may be used, and contact printing (ink jet etc) may be carried out in certain circumstances.

A further advantage of contactless printing is that no additional packaging work, for example, film wrapping, is required at the end user site prior to distribution.

Contactless printing may be carried out using any suitable method known in the art. Preferably, contactless printing is carried out via CO2, YAG or Fiber laser marking. The frequency of the laser may be such that there is no adverse reaction with or any damage to the plastic wrapping but one or more unique identifier may be applied to the receptacle, a label disposed on the receptacle, and/or an additional laser reactive label or substrate on the bottle for enhanced laser marking. This method may be carried out whilst the receptacle is held in position by the recess in the container. CO₂ laser marking is one of the methods which is particularly suitable, especially where the container contains polypropylene material.

In addition to personalising the sample bottle, the same process can be used to add other information, for example the name and postal address or other postal details, symbols or graphical information to the FIT pack for distribution. A method of combining the laser etching on the bottle and ink-jetting information eg a postal address label in process is possible. This laser capability may be added to new or existing mailing equipment to enable high throughput production.

One or more embodiments of the invention will now be described with reference to the accompanying figures, in which:
Figure 1 is a view from the front, one side and above of the container of the present invention in a fully open configuration.
Figure 2 is a view from one side of the container of the present invention in a partially open configuration.
Figure 3 is a view from the front, one side and above of the container depicted in claim 1, further depicting the optional fluid impermeable holder for housing a biological prior to insertion into the recess.
Figure 4 is a view from the rear, one side and below of the container of the present invention in a closed configuration.
Figure 5 is a view from the rear, one side and below of the container depicted in Figure 4.
Figure 6 is a view from the rear, one side and below of the container of the present invention in a broken configuration.
Figure 7 is a view from the rear, one side and below of the container of the present invention in an alternative broken configuration
Figure 8 is a view from above of a pre-cut and pre-creased blank erectable to form a container of the present invention.

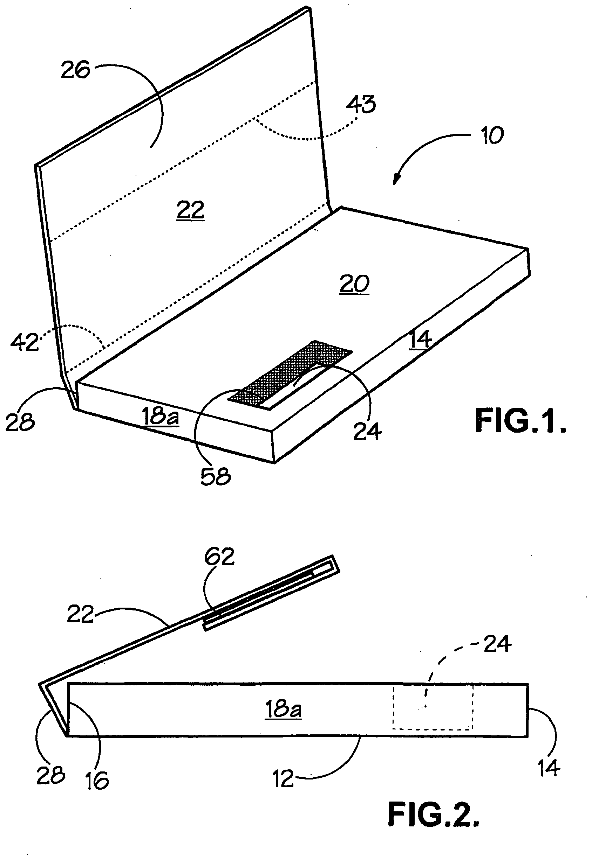

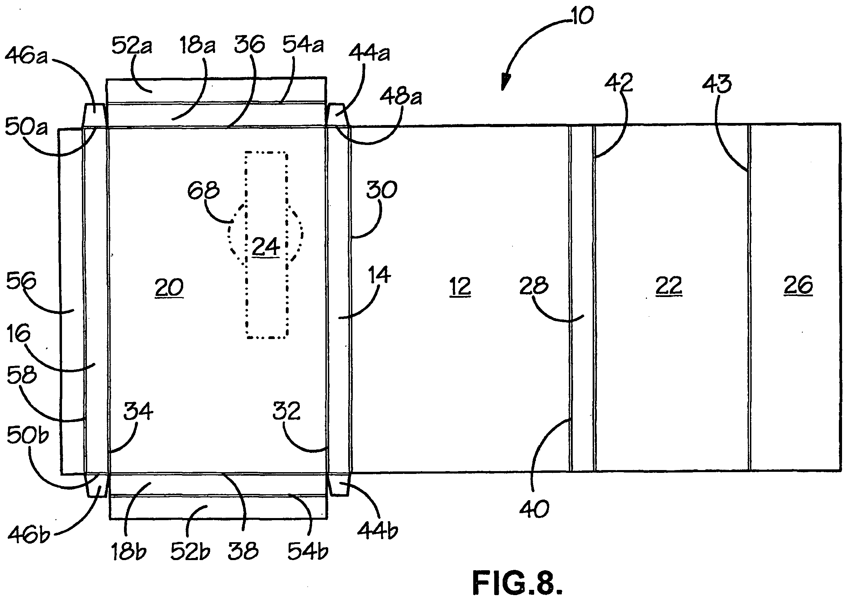

Referring to the figures, the fluid impermeable container 10 is formed from a sheet of laminated cardboard. It is generally cuboidal in shape, having a base 12, upright front 14, rear 16 and side walls 18a, 18b, an upper wall 20, and a closure flap 22. Upper wall 20 lies in a substantially identical plane to base 12, and defines an opening to a recess 24 for receiving a receptacle (not shown) containing a biological sample to be transported. Closure flap 22 has a sealing portion 26 which sealingly encloses the recess 24 when the container 10 is in a closed configuration. Referring to Figures 1 and 8, base 12, upper wall 20 and closure flap 22 are of substantially identical dimensions to one another and are all generally oblong in shape. Base 12 is separated from upper wall 20 along long edge 30 by front wall 14. Front wall 14 is separated from upper wall 20 by long edge 32, which lies opposite long edge 30. Rear wall 16 is of substantially identical dimensions to front wall 14 and is separated from upper wall 20 by long edge 34, which lies opposite long edge 32. Side walls 18a, 18b lie along opposite short edges 36, 38 respectively of upper wall 20. Base 12 is separated from closure flap 22 along long edge 40 by rear wall cover 28, which lies along an opposite edge of base 12 to front wall 14. Rear wall cover 28 is separated from closure flap 22 by long edge 42. Rear wall cover 28 is of substantially identical dimensions to front 14 and rear 16 wall, and lies substantially contiguous with rear wall 16 when container 10 is in a closed configuration. Sealing portion 26 is disposed towards an opposite edge of closure flap 22 to rear wall cover 28. Sealing portion 26 is distinguished from closure flap 22 by edge 43. Edges 30, 32, 34, 36, 38, 40, 42, 43 are pre-creased.

Still referring to Figures 1 and 8, front 14 and rear 16 walls each have a pair of adhesive tabs 44a, 44b and 46a, 46b respectively, extending outwardly from their respective shortest edges 48a, 48b, 50a, 50b. Each of side walls 18a, 18b also has an adhesive tab 52a, 52b respectively, each disposed along an opposite edge 54a, 54b respectively, to upper wall 20. Rear wall 16 has a further adhesive tab 56 disposed along an opposite edge 58 to upper wall 20. Edges 48a, 48b, 50a, 50b, 54a, 54b, 58 are pre-creased. During assembly of container 10, adhesive tabs 44a, 44b, 46a, 46b, 52a, 52b, 56 are secured to the inner surface of base 12 such that they are concealed from view. The performance of the adhesive is not compromised in the event of a spillage within container 10.

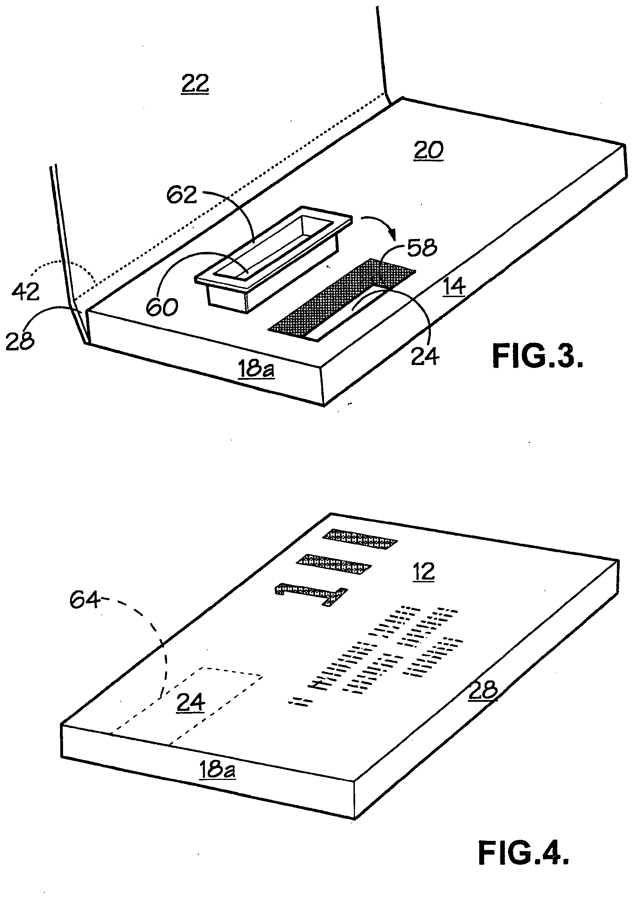

Turning now to Figures 1 and 3, container 10 comprises a plurality of container reinforcing members 58 provided as layers of corrugated cardboard. Reinforcing members 58 are of substantially identical dimensions to base 12 and upper wall 20, and are sandwiched therebetween when container 10 is erected from sheet material. Reinforcing members 58 have a cutout portion complementarily shaped to recess 24, such that portions of reinforcing members 58 form the side walls of recess 24. Reinforcing members 58 are secured in place once container 10 is erected from sheet material.

Referring to Figures 1 and 8, upper wall 20 also comprises semicircular cutaways 68 located along opposite long edges of the opening to recess 24. A portion of the reinforcing members 58 comprises complementarily shaped cutaway portions to facilitate access to a receptacle located inside recess 24.

Referring to Figure 3, a sample holder 60 in the form of a tray may be provided, the sample holder 60 being complementarily shaped to recess 24. Sample holder 60 is designed to receive a receptacle containing a biological sample and is therefore manufactured from a fluid impermeable material, more specifically plastic. Sample holder 60 further comprises a lip 62, the underside of which abuts, and is adhesively secured to, the upper surface of upper wall 20 when sample holder 60 is located in recess 24. An interior portion of sample holder 60 contains a cushioning and an absorbent material, such as cotton wool or the like.

Prior to use, and referring to Figure 2, container 10 adopts a partially open configuration in which sealing portion 26 of closure flap 22 is folded inwardly along edge 43, such that sealing portion 26 lies substantially contiguous with closure flap 22. Sealing portion 26, which is generally formed from double sided adhesive tape, is prevented from adhering to closure flap 22 by backing strip 62. An empty receptacle for receiving a biological sample may then be located in recess 24 and a recipient's address, or other information, may be printed onto the uppermost surface of closure flap 22, before an outer wrapper, such as transparent cellophane is applied. Upon receipt of container 10 in its partially open configuration, recess 24 and a receptacle contained therein are presented to a user.

In use, and referring to the Figures, container 10 is exposed by removal, if present, of an outer wrapper, thereby allowing container 10 to adopt a fully open configuration in which sealing portion 26 is folded outwardly along edge 43 to reveal user instructions printed onto the inner surface of closure flap 22. A user then removes the receptacle from recess 24 and inserts into it a sample of biological material. The receptacle is then sealed and relocated back into recess 24. Backing strip 62 is then removed from sealing portion 26 and discarded. Next, sealing portion 26 is lowered onto the opening to recess 24, such that the upper boundaries of recess 24 and cutaways 68 on upper wall 20 are fully and sealingly enclosed by sealing portion 26. The adhesive and sealing characteristics of sealing portion 26 are not compromised by spillages within recess 24.

Accordingly, when used as a return mailing article, container 10, being in a partially open configuration, arrives with a user in a transparent wrapper, with recess 24, and a receptacle contained therein, clearly visible. Container 10 is then unwrapped, a biological sample is introduced into the receptacle, and the receptacle then relocated back into recess 24. Container 10 is then sealingly closed by the user according to the above protocol, and conveniently mailed to a recipient, normally a laboratory or other medical institute, whose address may have been pre-printed on container 10.

In its closed configuration, container 10 complies with specific regulations governing the transportation of biological material, such as those imposed by UN3373. Container 10 is suitably sized so as to be postable through a standard letter box, and addresses or other relevant information may be printed on the outer surface of either closure flap 22 or base 12.

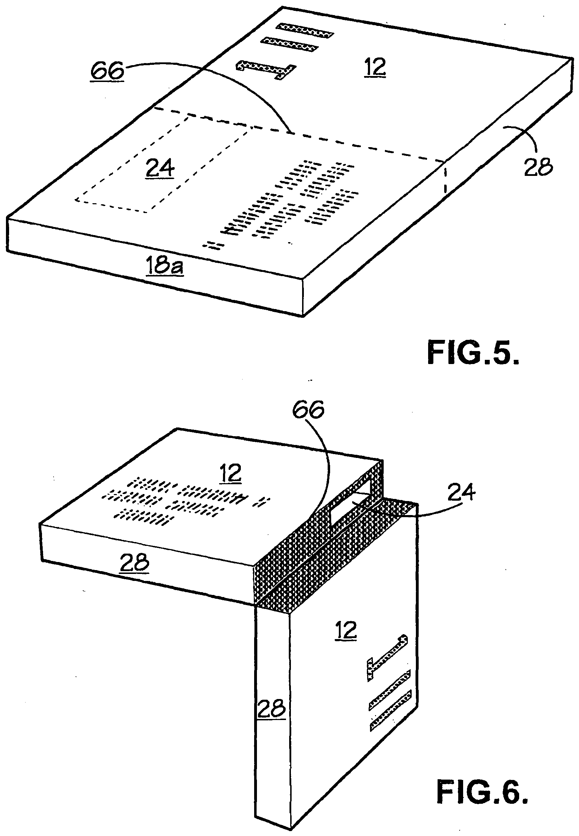

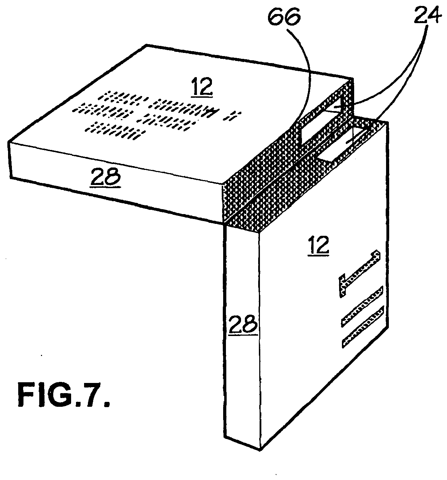

Once at its destination, most probably a hospital, laboratory or other medical institute, the sample of biological material must be retrieved from container 10. Referring to Figure 4, container 10 is provided with a perforated release tab 64 formed into base 12 and located over the underside of recess 24. Release tab 64 may therefore be torn away to expose a portion of recess 24 in which the receptacle containing the biological sample is contained. Alternatively, and referring to Figures 5 and 6, the sample of biological material may be retrieved by tearing along a perforation 66 that extends through a substantially median point of container 10. Perforation 66 intersects a face of recess 24, such that tearing container 10 along perforation 66 exposes a portion of recess 24, thereby allowing the sample to be retrieved. Alternatively, and referring to Figure 7, perforation 66 may intersect recess 24 at any point along its length, such that, upon tearing, the receptacle containing the biological sample protrudes therefrom and is easily retrievable.

While specific embodiments have been described herein for the purpose of reference and illustration, various modifications will be apparent to a person skilled in the relevant art and may be made without departing from the scope of the invention as defined by the appended claims. For example, container 10 may be formed from a number of other sheet materials, such as certain plastics, in which case further fluid impermeability characteristics may be conferred to container 10. The shape of container 10 may also differ from that described herein, and various other three dimensional forms are equally envisageable. Reinforcing members 58 may be constructed from materials other than cardboard, such as rigid foams or sponges. The reinforcing members may be impregnated or dispersed with a material having absorbency characteristics, thereby minimising even further the risk of spillage. Recess 24 may be shaped to receive any number of conventional specimen receptacles. Cutaways 68 may be one or more in number, and may be of any size of shape. Where sample holder 60 is not used, recess 24 may be directly packed with one or more cushioning or absorbent materials, including fabrics, foams, sponges, pads and powders. Sealing portion 26 may comprise adhesive means other than double sided adhesive tape, such as any number of glues. The adhesives are preferably selected such that their performance is not compromised in the event of a spillage. Release tab 64 may be alternatively located to expose any accessible face of recess 24. Release tab 64 may also be located on closure flap 22, providing that the sealing characteristics of container 10 are not compromised. Perforation 66 may be relocated so as to expose, upon tearing, one or more other portions of recess 24.

## Claims

1. A method for manufacturing a fluid-impermeable container containing a receptacle for transporting a biological sample, comprising the steps of:
a. folding a blank to form the container (10), the container (10) comprising folded sheet material, the folded sheet material being fluid impermeable and defining a base (12), enclosing side walls (18a, 18b), an upper wall (20), and a container closing member (22), the upper wall (20) being configured to define an opening to a recess (24) for receiving a receptacle containing a biological sample to be transported, wherein the recess (24) is sealingly enclosed by the container closing member (22) when the container (10) is in a closed configuration;
b. inserting a receptacle into the container (10);
characterised int that the method comprises further the steps of :
c. applying a transparent plastic film around the container and receptacle; and
d. applying a unique identifier to the receptacle and/or container through the film by contactless printing, wherein the unique identifier is one or more item of information which is specific to the source of the sample.

2. A method for manufacturing a container as claimed in claim 1, wherein the sheet material is rigid and, optionally, comprises cardboard.

3. A method for manufacturing a container as claimed in claim 2, wherein the container (10) is sized to be postable through a standard letter box.

4. A method for manufacturing a container as claimed in claim 1, 2 or 3, wherein the folded sheet material comprises a fluid impermeable coating and optionally, wherein the fluid impermeable coating is provided as a laminate coating on the sheet material.

5. A method for manufacturing a container as claimed in any preceding claim, wherein the container closing member (22) comprises an adhesive portion (26).

6. A method for manufacturing a container as claimed in claim 5, wherein the adhesive portion (26) does not extend over an area of the container closing member (22) that overlies the opening to the recess (24) when the container (10) is in a closed configuration and optionally, wherein the adhesive is exposed by releasing a backing strip.

7. A method for manufacturing a container as claimed in any preceding claim, further comprising one or more container reinforcing members (58) and optionally, wherein the one or more container reinforcing members (58) comprises an absorbent and/or wherein the container reinforcing member (58) comprises one or more Inserts, around which the sheet material is folded and optionally, wherein the one or more inserts are made from a foam or from corrugated cardboard.

8. A method for manufacturing a container as claimed in any preceding claim, wherein the recess (24) comprises a cushioning material and/or wherein the recess (24) comprises an absorbent material and optionally, wherein the quantity of absorbent material is sufficient to absorb all of the biological sample to be transported and optionally, wherein the absorbent material is a foam or cotton wool.

9. A method for manufacturing a container as claimed in any preceding claim, wherein the recess (24) further comprises a fluid Impermeable holder (60) for housing the biological sample to be transported and optionally, wherein the holder (60) is removable and/or wherein the holder (60) is made from plastic and optionally, wherein the recess (24) further comprises one or more cutaway portions (68) for facilitating access to the recess (24).

10. A method for manufacturing a container as claimed in any preceding claim, wherein the recess (24) is substantially larger than the receptacle which it is intended to receive and optionally, wherein the recess (24) further comprises receptacle retaining means for retaining the receptacle in a given part of the recess (24) and optionally, wherein the receptacle retaining means comprises a constricted portion.

11. A method for manufacturing a container as claimed in any preceding claim, further comprising sample releasing means for accessing the recess (24) other than via the container closing member (22).

12. A method for manufacturing a container as claimed in claim 11, wherein the sample releasing means comprises a perforated release tab (64) complementarily shaped to at least one of the faces of the recess (24) and optionally, wherein the perforated release tab (64) is disposed on the base (12) of the container (10).

13. A method for manufacturing a container as claimed in claim 11, wherein the sample releasing means comprises a line of weakness extending through the container (10) and intersecting the recess (24) along one of its faces or wherein the sample releasing means comprises a line of weakness extending through the container (10) and intersecting the recess (24) at any point along its length.

14. A method for manufacturing a container as claimed in any one of the preceding claims wherein the container (10) has a unique identifier disposed thereon or therein and optionally, wherein the unique identifier is applied using contactless printing by more than one type of printing and optionally, wherein the contactless printing is achieved by any one or more of the following: CO₂, YAG or Fiber laser marking.

15. A method for manufacturing a container as claimed in any of the preceding claims, wherein the unique identifier is the identity of a specific human or animal patient and optionally, wherein the unique identifier comprises any one or more of the following: readable patient data, such as, name, date, health record number, kit number and a 1D and/or 2D barcode that also encompasses all or part of the above.

## Patentansprüche

1. Verfahren zum Anfertigen eines fluidundurchlässigen Behälters, der ein Gefäß zum Transportieren einer biologischen Probe enthält, umfassend folgende Schritte:
a. Falten eines Rohlings zum Bilden des Behälters (10), wobei der Behälter (10) gefaltetes flächiges Material umfasst, wobei das gefaltete flächige Material fluidundurchlässig ist und einen Boden (12), umschließende Seitenwände (18a, 18b), eine obere Wand (20) und ein Behälterverschlusselement (22) definiert, wobei die obere Wand (20) dazu konfiguriert ist, eine Öffnung zu einer Aussparung (24) zum Aufnehmen eines eine zu transportierenden biologische Probe enthaltenden Gefäßes zu definieren, wobei die Aussparung (24) von dem Behälterverschlusselement (22) dichtend umschlossen wird, wenn sich der Behälter (10) in einer geschlossenen Konfiguration befindet;
b. Einsetzen eines Gefäßes in den Behälter (10);
**dadurch gekennzeichnet, dass** das Verfahren weiter folgende Schritte umfasst:
c. Aufbringen einer durchsichtigen Kunststofffolie um den Behälter und das Gefäß; und
d. Aufbringen einer eindeutigen Kennzeichnung auf das Gefäß und/oder den Behälter mittels berührungslosen Druckens durch die Folie, wobei es sich bei der eindeutigen Kennzeichnung um eine oder mehrere für die Herkunft der Probe spezifische Einzelinformationen handelt.

2. Verfahren zum Anfertigen eines Behälters nach Anspruch 1, wobei das flächige Material starr ist und optional Karton umfasst.

3. Verfahren zum Anfertigen eines Behälters nach Anspruch 2, wobei der Behälter (10) bemessen ist, um durch einen gewöhnlichen Briefkasten gesteckt werden zu können.

4. Verfahren zum Anfertigen eines Behälters nach Anspruch 1, 2 oder 3, wobei das gefaltete flächige Material eine fluidundurchlässige Beschichtung umfasst und wobei optional die fluidundurchlässige Beschichtung als eine Laminatbeschichtung auf dem flächigen Material bereitgestellt ist.

5. Verfahren zum Anfertigen eines Behälters nach einem der vorangehenden Ansprüche, wobei das Behälterverschlusselement (22) einen Klebeabschnitt (26) umfasst.

6. Verfahren zum Anfertigen eines Behälters nach Anspruch 5, wobei sich der Klebeabschnitt (26) nicht über einen Bereich des Behälterverschlusselements (22) erstreckt, der über der Öffnung zu der Aussparung (24) liegt, wenn sich der Behälter (10) in einer geschlossenen Konfiguration befindet, und wobei optional der Klebstoff durch Ablösen eines Abdeckstreifens freigelegt wird.

7. Verfahren zum Anfertigen eines Behälters nach einem der vorangehenden Ansprüche, weiter umfassend ein oder mehrere Behälterversteifungselemente (58) und wobei optional das eine oder die mehreren Behälterversteifungselemente (58) ein Absorptionsmittel umfasst und/oder wobei das Behälterversteifungselement (58) einen oder mehrere Einsätze umfasst, um die das flächige Material gefaltet wird, und wobei optional der eine oder die mehreren Einsätze aus einem Schaumstoff oder aus Wellkarton hergestellt sind.

8. Verfahren zum Anfertigen eines Behälters nach einem der vorangehenden Ansprüche, wobei die Aussparung (24) ein Polstermaterial umfasst und/oder wobei die Aussparung (24) ein saugfähiges Material umfasst und wobei optional die Menge an saugfähigem Material ausreicht, um die gesamte zu transportierende biologische Probe aufzusaugen, und wobei es sich optional bei dem saugfähigen Material um einen Schaumstoff oder Watte handelt.

9. Verfahren zum Anfertigen eines Behälters nach einem der vorangehenden Ansprüche, wobei die Aussparung (24) weiter einen fluidundurchlässigen Halter (60) zum Unterbringen der zu transportierenden biologischen Probe umfasst und wobei optional der Halter (60) herausnehmbar ist und/oder wobei der Halter (60) aus Kunststoff hergestellt ist und wobei optional die Aussparung (24) weiter einen oder mehrere ausgeschnittene Abschnitte (68) zum Erleichtern des Zugriffs auf die Aussparung (24) umfasst.

10. Verfahren zum Anfertigen eines Behälters nach einem der vorangehenden Ansprüche, wobei die Aussparung (24) wesentlich größer ist als das Gefäß, die sie aufnehmen soll, und wobei optional die Aussparung (24) weiter ein Gefäßsicherungsmittel zum Sichern des Gefäßes in einem gegebenen Teil der Aussparung (24) umfasst und wobei optional das Gefäßsicherungsmittel einen verengten Abschnitt umfasst.

11. Verfahren zum Anfertigen eines Behälters nach einem der vorangehenden Ansprüche, weiter umfassend ein Probenfreigabemittel zum anderen Zugreifen auf die Aussparung (24) als über das Behälterverschlusselement (22).

12. Verfahren zum Anfertigen eines Behälters nach Anspruch 11, wobei das Probenfreigabemittel eine perforierte Freigabelasche (64) umfasst, die komplementär zu mindestens einer der Seiten der Aussparung (24) geformt ist und wobei optional die perforierte Freigabelasche (64) an dem Boden (12) des Behälters (10) angeordnet ist.

13. Verfahren zum Anfertigen eines Behälters nach Anspruch 11, wobei das Probenfreigabemittel eine Schwächelinie umfasst, die sich durch den Behälter (10) erstreckt und die Aussparung (24) entlang einer ihrer Seiten kreuzt oder wobei das Probenfreigabemittel eine Schwächelinie umfasst, die sich durch den Behälter (10) erstreckt und die Aussparung (24) an einer beliebigen Stelle entlang ihrer Länge kreuzt.

14. Verfahren zum Anfertigen eines Behälters nach einem der vorangehenden Ansprüche, wobei der Behälter (10) eine darauf oder darin angeordnete eindeutige Kennzeichnung aufweist und wobei optional die eindeutige Kennzeichnung unter Verwendung von berührungslosem Drucken mittels mehr als einer Art von Drucken aufgebracht wird und wobei optional das berührungslose Drucken mittels eines oder mehrerer der Folgenden erreicht wird: CO₂-, YAG- oder Faserlaserbeschriftung.

15. Verfahren zum Anfertigen eines Behälters nach einem der vorangehenden Ansprüche, wobei es sich bei der eindeutigen Kennzeichnung um die Identität eines bestimmten menschlichen oder tierischen Patienten handelt und wobei optional die eindeutige Kennzeichnung eines oder mehrere der Folgenden umfasst: lesbare Patientendaten, wie etwa Name, Datum, Gesundheitsaktenzeichen, Kit-Nummer und einen 1D- und/oder 2D-Barcode, der ebenfalls alle oder einen Teil der Vorangehenden einschließt.

## Revendications

1. Procédé de fabrication d'un récipient imperméable aux fluides contenant un réceptacle servant à transporter un échantillon biologique, comportant les étapes consistant à :
a. plier une découpe pour former le récipient (10), le récipient (10) comportant un matériau en feuille replié, le matériau en feuille replié étant imperméable aux fluides et définissant une base (12), des parois latérales enveloppantes (18a, 18b), une paroi supérieure (20), et un élément de fermeture de récipient (22), la paroi supérieure (20) étant configurée pour définir une ouverture donnant sur un évidement (24) servant à recevoir un réceptacle contenant un échantillon biologique destiné à être transporté, dans lequel l'évidement (24) est enfermé de manière étanche par l'élément de fermeture de récipient (22) quand le récipient (10) est dans une configuration fermée ;
b. insérer un réceptacle dans le récipient (10) ;
**caractérisé en ce que** le procédé comporte par ailleurs les étapes consistant à :
c. appliquer un film plastique transparent autour du récipient et du réceptacle ; et
d. appliquer un identifiant unique sur le réceptacle et/ou le récipient au travers du film par impression sans contact, dans lequel l'identifiant unique est un ou plusieurs éléments d'informations qui sont spécifiques à la source de l'échantillon.

2. Procédé de fabrication d'un récipient selon la revendication 1, dans lequel le matériau en feuille est rigide et, éventuellement, comporte du carton.

3. Procédé de fabrication d'un récipient selon la revendication 2, dans lequel le récipient (10) est dimensionné pour être en mesure d'être posté au travers d'une boîte aux lettres de taille normale.

4. Procédé de fabrication d'un récipient selon la revendication 1, la revendication 2 ou la revendication 3, dans lequel le matériau en feuille replié comporte un revêtement imperméable aux fluides et éventuellement, dans lequel le revêtement imperméable aux fluides est mis en oeuvre sous la forme d'un revêtement stratifié sur le matériau en feuille.

5. Procédé de fabrication d'un récipient selon l'une quelconque des revendications précédentes, dans lequel l'élément de fermeture de récipient (22) comporte une partie adhésive (26).

6. Procédé de fabrication d'un récipient selon la revendication 5, dans lequel la partie adhésive (26) ne s'étend pas sur une zone de l'élément de fermeture de récipient (22) qui recouvre l'ouverture donnant sur l'évidement (24) quand le récipient (10) est dans une configuration fermée et éventuellement, dans lequel l'adhésif est exposé par libération d'une bande de soutien.

7. Procédé de fabrication d'un récipient selon l'une quelconque des revendications précédentes, comportant par ailleurs un ou plusieurs éléments de renfort de récipient (58) et éventuellement, dans lequel lesdits un ou plusieurs éléments de renfort de récipient (58) comportent un absorbant et/ou dans lequel l'élément de renfort de récipient (58) comporte une ou plusieurs pièces rapportées, autour desquelles le matériau en feuille est plié et éventuellement, dans lequel lesdites une ou plusieurs pièces rapportées sont réalisées à partir d'une mousse ou à partir d'un carton ondulé.

8. Procédé de fabrication d'un récipient selon l'une quelconque des revendications précédentes, dans lequel l'évidement (24) comporte un matériau de bourrage et/ou dans lequel l'évidement (24) comporte un matériau absorbant et éventuellement, dans lequel la quantité de matériau absorbant est suffisante pour absorber l'intégralité de l'échantillon biologique devant être transporté et éventuellement, dans lequel le matériau absorbant est une mousse ou du coton hydrophile.

9. Procédé de fabrication d'un récipient selon l'une quelconque des revendications précédentes, dans lequel l'évidement (24) comporte par ailleurs un support imperméable aux fluides (60) servant à recevoir l'échantillon biologique destiné à être transporté et éventuellement, dans lequel le support (60) est amovible et/ou dans lequel le support (60) est réalisé en plastique et éventuellement, dans lequel l'évidement (24) comporte par ailleurs une ou plusieurs parties découpées (68) servant à faciliter l'accès à l'évidement (24).

10. Procédé de fabrication d'un récipient selon l'une quelconque des revendications précédentes, dans lequel l'évidement (24) est sensiblement plus grand que le réceptacle qu'il est destiné à recevoir et éventuellement, dans lequel l'évidement (24) comporte par ailleurs un moyen de retenue de réceptacle servant à retenir le réceptacle dans une partie donnée de l'évidement (24) et éventuellement, dans lequel le moyen de retenue de réceptacle comporte une partie à étranglement.

11. Procédé de fabrication d'un récipient selon l'une quelconque des revendications précédentes, comportant par ailleurs un moyen de libération d'échantillon servant à accéder à l'évidement (24) autre que par le biais de l'élément de fermeture de récipient (22).

12. Procédé de fabrication d'un récipient selon la revendication 11, dans lequel le moyen de libération d'échantillon comporte une languette de libération perforée (64) de forme complémentaire par rapport à au moins l'une des faces de l'évidement (24) et éventuellement, dans lequel la languette de libération perforée (64) est disposée sur la base (12) du récipient (10).

13. Procédé de fabrication d'un récipient selon la revendication 11, dans lequel le moyen de libération d'échantillon comporte une ligne de moindre résistance s'étendant au travers du récipient (10) et croisant l'évidement (24) le long de l'une de ses faces ou dans lequel le moyen de libération d'échantillon comporte une ligne de moindre résistance s'étendant au travers du récipient (10) et croisant l'évidement (24) en tout point quelconque le long de sa longueur.

14. Procédé de fabrication d'un récipient selon l'une quelconque des revendications précédentes, dans lequel le récipient (10) a un identifiant unique disposé sur celui-ci ou dans celui-ci et éventuellement, dans lequel l'identifiant unique est appliqué par impression sans contact par plus d'un type d'impression et éventuellement, dans lequel l'impression sans contact est réalisée par l'un quelconque ou plusieurs parmi les suivants : le marquage au laser à dioxyde de carbone, au grenat d'yttrium et d'aluminium ou à fibre.

15. Procédé de fabrication d'un récipient selon l'une quelconque des revendications précédentes, dans lequel l'identifiant unique est l'identité d'un patient humain ou animal spécifique et éventuellement, dans lequel l'identifiant unique comporte l'un quelconque ou plusieurs parmi les suivants : des données lisibles se rapportant au patient, telles que le nom, la date, le numéro de dossier médical, le numéro de trousse et un code à barres 1D et/ou 2D qui comprend également tous ou une partie des éléments ci-dessus.
